# NEUE EUROPÄISCHE PATENTSCHRIFT

(11) **EP 1 921 939 B2**
(45) Veröffentlichungstag und Bekanntmachung des Hinweises auf die Entscheidung über den Einspruch: **16.09.2015**
(45) Hinweis auf die Patenterteilung: 22.06.2011
(21) Anmeldenummer: 06805658.9
(22) Anmeldetag: 06.09.2006
(51) Int. Cl.: A43B 7/08, A43B 7/12, A41D 27/28, A61F 9/02, A42B 3/28, A63B 71/12

(54) **BELÜFTUNGSEINSATZ**
VENTILATION INSERT
INSERT D'AERATION

(30) Priorität: 06.09.2005 DE 102005043069; 21.10.2005 DE 102005051575
(43) Veröffentlichungstag der Anmeldung: 21.05.2008
(73) Patentinhaber: IQTEX Patentverwaltung UG (haftungsbeschränkt), 20354 Hamburg (DE)
(72) Erfinder: Dehn, Michael Christian, 21614 Buxtehude (DE)
(74) Vertreter: Kossak, Sabine
(86) Internationale Anmeldenummer: PCT/EP2006/008691
(87) Internationale Veröffentlichungsnummer: WO 2007/028594

(56) Entgegenhaltungen:
- EP-A2- 1 054 095
- EP-A2- 1 054 095
- WO-A-2005/095692
- DE-A1- 19 619 858
- DE-U1-202004 000 307
- DE-U1-202004 000 307
- JP-A- H05 220 004
- JP-A- H07 136 002
- JP-A- H10 229 902
- JP-A- H11 222 710
- JP-A- 2005 102 933
- US-A- 3 607 591
- US-A1- 2004 049 942
- DIAPLEX: "What's So Intelligent" INTERNET ARTICLE, [Online] XP002409993 Gefunden im Internet: URL:http://web.archive.org/web/19991202002 110/http://www.diaplex.com/intelligent.htm l> [gefunden am 1999-12-02]

## Beschreibung

Die Erfindung betrifft einen Belüftungseinsatz für die Belüftung von Textilien, Schuhen sowie Schuhsohlen, Handschuhen, Schutzhelmen und deren Visieren, Schutzbrillen, Isoliermatten, Polstermatten, Knie- und Schienenbeinschützer, Kopfbedeckungen und ähnlichen Artikeln.

Bisher bekannte Belüftungen für die vorgenannten Artikel verwenden im wesentlichen. Mikrofasern, offenporige Materialien oder Belüftungsöffnungen.

Öffnungen oder offenporige Materialien bieten nach wie vor die effektivste Möglichkeit für einen Luftaustausch sowie der damit verbundenen Abführung von Wasserdampf und Wärme. Öffnungen oder Materialdurchbrüche, zum Beispiel an Jacken, weisen bei einigen Lösungen Verschlussmöglichkeiten wie Reiß- oder Klettverschlüsse auf, die nach einem manuellen Verschließen oder Öffnen ein Eindringen von Wasser verhindern oder eine gezielte Belüftung ermöglichen. Des Weiteren besteht die Möglichkeit in der Verwendung von Faserschichten, mit denen Öffnungen dauerhaft abgedeckt werden und so ein Eindringen von Wasser verhindern.

Schuhe weisen oftmals im Bereich des Schuhoberteils Öffnungen auf, die zum Teil mit Strukturen, zum Beispiel Lamellen, in Verbindung stehen die eine verbesserte Zuführung von Außenluft bzw. ein nach Aussendringen von Wasserdampf ermöglichen sollen. Manche Schuhe verfügen über Öffnungen in den Schuhsohlen. Um hier ein Eindringen von Wasser und Fremdkörpern zu vermeiden, werden diese Öffnungen beispielsweise mit einer Spezialmembran oder einer Faserschicht abgedeckt.

Spezialmembranen oder Faserschichten, mit denen Belüftungsöffnungen vor dem Eindringen von Wasser und Fremdkörpern geschützt werden sollen, behindern im erheblichen Maße eine Luftzirkulation und somit einen Abtransport von verbrauchter Luft bzw. von Wasserdampf. Bei der Verwendung von Spezialmembranen handelt es sich um Laborausführungen, die zumeist keinen tatsächlich spürbaren Erfolg mit sich bringen. Zusätzlich weisen Materialien dieser Art den Nachteil auf, dass sie nur einseitig durchlässig sind, das heißt, wenn Wasserdampf nach außen dringen soll, ist es aufgrund der Materialbeschaffenheit, nämlich der Ausrichtung der Kapillaren, nicht möglich, dass Außenluft nach innen dringt.

Es sind bereits Lösungsversuche bekannt, nach denen Fasern textiler Flächen mit einem Elastomer (so genannte Superabsorber) beschichtet oder imprägniert werden, so dass sie bei Berührung mit Wasser aufquellen und durch eine flüssigkeitsbedingte Volumenzunahme Zwischenräume, welche sich zwischen den Fasern befinden, abdichten und so das Eindringen von Wasser verhindern. Die Verwendung von Superabsorbern in Textilflächen erweist sich in der Form als nachteilig, als dass sich entsprechende Textilien ganzftächig mit Wasser vollsaugen, was eine starke Gewichtzunahme zur Folge hat. Außerdem besteht der Nachteil, dans Superabsorber nicht dauerhaft mit textilen Fasern verbunden werden können, dass sie nicht auf Dauer abdichten und dass entsprechende Textilien nach einer Feuchtigkeitsaufnahme eine oft als unangenehm empfunden, geleeartige Konsistenz aufweisen.

Die nach wie vor effektivste Möglichkeit der Belüftung besteht in der Verwendung offenporiger Materialien oder Öffnungen. Verschiedene Öffnungen in Textilien und Schuhen bieten die Möglichkeit, dass sie durch Abdeckelemente per Hand vor einem Wassereintritt geschützt werden können. Entsprechende Abdeckelemente werden vorzugsweise durch Reiß- oder Klettverschlüsse in Ihrer Position befestigt oder sie sind unverlierbarerer Bestandteil von Textilien oder Schuhen und werden durch Schwenk- oder Schiebebewegung in ihrer Position verändert..

Diese vorgenannten Lösungen der Belüftungsöffnungen weisen jedoch Nachteile in der Form auf, dass Wasser eindringen kann, dass sie nur unzureichend eine Belüftung ermöglichen oder sie per Hand vor dem Eindringen von Flüssigkeiten geschützt werden müssen.

Ein Belüftungseinsatz für die Belüftung von Schuhen ist aus DE 20 2004 307 bekannt.

In der Bauindustrie werden ebenfalls Superabsorber benutzt, um Leckagen abzudichten, u. a. bei Tiefseekabeln und Dachabdichtungen. Hierbei geht es aber nur um eine Abdichtung gegen das Eindringen von Flüssigkeiten und nicht um den Schutz von Belüftungsöffnungen.

Faserschichten, die durch Ihre Feuchtigkeitsaufnahme ein Eindringen von Wasser verhindern, haben den Nachteil, dass sie durch Ihre relativ starke Dichte und Materialstärke nur im sehr reduzierten Maße luftdurchlässig sind, zusätzlich können sie aufgrund ihrer Kapillaren Wasser an das Innere weiterleiten (Feuchtigkeitsbrücke). Sie brauchen daher lange bis sie wieder trocknen, Schimmel- und Bakterienbildung sind möglich.

Um ein Eindringen von Wasser und Schmutz zu verhindern, muss deshalb bislang ein Kompromiss zwischen Tragekomfort und Funktion eingegangen werden.

Der Erfindung liegt nun die Aufgabe zugrunde, einen Belüftungseinsatz mit Belüftungsöffnungen zu schaffen, die sich bei Nässe oder niedrigen Temperaturen selbständig verschließen und so ein Eindringen von Wasser oder kalter Luft verhindern, und die schnell trocknen und sich beim Trocknen oder steigenden Temperaturen wieder öffnen und eine gezielte Belüftung herbeiführen. Dabei sollen derartige Belüftungseinsätze auf baulich einfache Weise, kostengünstig und aus gesundheitlich unbedenklichen Materialien hergestellt werden können.

Die Aufgabe wird erfindungsgemäß gelöst durch einen Belüftungseinsatz gemäß Patentanspruch 1. Weitere Ausgestaltungen sind Gegenstand der Unteransprüche oder nachfolgend beschrieben.

Das quellfähige Material kann ein Granulat oder Stücken von Flachmaterial oder ein fadenförmig geformtes Material sein. Es kann sich um einen Flüssigkeitsabsorber, bevorzugt einen granulatförmigen Flüssigkeitsabsorber aus vernetztem Natriumpolyacrylat, ein Polyacrylsäurecopolymer, ein Eiweiß oder Kasein handeln. Ein bei Feuchtigkeit quellfähiges Thermoplast-Elastomer-Composite-Material ist zum Beispiel unter der Bezeichnung Q-TE-C erhältlich (Fraunhofer-Institut). Als Material, das durch Wärme oder elektrische Spannung seine Form ändert, können Formgedächtnislegierungen oder Thermoplast-Elastomer-Composites verwendet werden.

Die Trägerschicht ist ein Vlies-Material, auf das das quellfähige Material aufgebracht ist.

Die Abdeckung ist ebenfalls ein Vlies-Material oder ein Kunststoff- oder Metallgitter.

Zur Verstärkung der Öffnungs- oder Schließwirkung kann die Anordnung mehrfach übereinander aufgebaut sein.

Die Abdeckung kann gleichzeitig als Verdunstungs- bzw. Zuführungsfläche für Feuchtigkeit dienen.

Ausgenutzt wird mit der Erfindung zum Beispiel ein durch Feuchtigkeit verursachtes reversibles Quellverhalten von Flüssigkeitsabsorbern wie zum Beispiel vernetztes Natriumpolyacrylat, Eiweißstoffen oder Kasein, um Lüftungsöffnungen selbsttätig zu verschließen oder zu öffnen, wobei es in der Lage ist, den Bewegungsablauf automatisch umzukehren. Es wird das Quell- bzw. Schrumpfverhalten von Absorbermaterialien genutzt, um einfache mechanische Bewegungen und damit eine Ventilwirkung hervorzurufen.

Als quellfähiges Material kommen auch Polymere mit der Fähigkeit der reversiblen Phasenumwandlung in Frage. Die Zwischenräume innerhalb dieses Materials, das als Vlies oder Fasermaterial vorliegen kann oder als Granulat auf solche Materialien aufgebracht ist, werden durch die Reaktion solcher Gedächtnislegierungen, ausgelöst durch Feuchtigkeit, Wärme oder eine elektrische Spannung, mindestens teilweise geschlossen. Durch ein wärmeempfindliches Polymer lässt sich beispielsweise ein selbstregulierender Belüftungseinsatz herstellen, der bei niedriger Außentemperatur selbsttätig die Belüftungsöffnungen verschließt oder bei hohen Temperaturen öffnet. Geeignete Kunststoffe sind hierfür bereits verfügbar, insbesondere Kunststoffe, die über einen sogenannte "Shape Memory Effekt" verfügen.

Nach einer ersten nicht erfindungsgemäßen Variante ist vorgesehen, dass sich Abdeckelemente, welche an einem ihrer Enden zum Beispiel durch Verkleben oder durch thermoplastisches Verschweißen mit der Trägerschicht derart verbunden sind, dass sie durch die Quell- oder Schrumpfwirkung eines mit der Trägerschicht oder den Abdeckelementen verbundenen quellfähigen Materials nach Art eines Scharniers klappbar sind und damit, je nach ausgeführter Richtung der Bewegung, Belüftungsöffnungen in der Abdeckung oder die Oberfläche einer luft- und dampfdurchlässigen Faserschicht freigeben oder abdecken.

Im Bereich des Scharniers befindet sich, beispielsweise als Granulat eingebracht z.B. in eine flüssigkeitsdurchlässige elastische Hülle, ein Dampf- und Flüssigkeitsabsorber wie zum Beispiel vernetztes Natriumpolyacrylat oder ein Thermoplast-Elastomer-Composite-Material, das auch als geformtes Material vorliegen kann. Im Bereich des Scharniers befinden sich die Lüftungsöffnungen oder dampfdurchlässige Faserschichten, die ein Eindringen von Flüssigkeit und ein schnelles nach außen Dringen von Wasserdampf ermöglichen. Dringt Nässe durch die Hülle die den Flüssigkeits-/Dampfabsorber umgibt, welche ein Verlieren des Dampfabsorbers verhindert, nimmt der Dampfabsorber die Nässe auf. Bedingt durch die Flüssigkeitsaufnahme und die damit verbundene Quellkraft (mindestens 50g pro m² cm) hebt oder senkt sich, je nach Ausführung, das Abdeckelement. Befindet sich der Dampfabsorber oberhalb des Scharniers, wird bei einer Flüssigkeitsaufnahme das Abdeckelement nach unten gedrückt. Befindet sich hingegen der Dampfabsorber auf der Unterseite des Abdeckelementes, hebt sich bei einer Flüssigkeitsaufnahme das Abdeckelement. Je nach Ausführung kann eine Belüftungsöffnung so durch die Quellkräfte geöffnet oder verschlossen werden. Durch Verdunsten der aufgenommenen Flüssigkeit reduziert sich das Volumen des Dampfabsorbers wieder, der Bewegungsablauf kehrt sich durch die Rückstellkräfte des Abdeckelementes, der elastischen Hülle, der Schrumpfung des Absorbers und/oder der des Scharniers um. Ein Verlieren des Flüssigkeits-/Dampfabsorber kann auch durch Halterungen verhindert werden.

An einem Rahmen der Trägerschicht und/oder Abdeckung können Befestigungen wie beispielsweise Verbindungsflächen, Klebe- oder Klettverschlussflächen oder Nähte auf- bzw. angebracht sein, die es ermöglichen, den Belüftungseinsatz über Materialdurchbrüche oder Öffnungen in dem zu belüftenden Gegenstand in einer gewünschten Position zu halten. Die Herstellung der Verbindungsflächen kann in unterschiedlichen Stärken erfolgen, je nach Anwendungsbereich. Die Flächen sind bevorzugt dünn und laufen nach außen hin auf 0 zusammen. Bevorzugt können die Verbindungsflächen bereits während der Spritzgussherstellung texturiert werden oder nachträglich durch mechanisches Aufrauen, Ätzen, Bestrahlen mit UV-Licht oder Behandlung mit Gasen, wie Ozon texturiert werden. Eine entsprechend texturierte Oberfläche der Verbindungsflächen erleichtert die Befestigung am zu belüftenden Objekt, wenn Verbindungsweisen wie Kleben oder Verschweißen oder Ähnliches gewählt werden. Der Rahmen kann auch zum thermoplastischen Verschweißen mit dem zu belüftenden Gegenstand dienen.

Nach einer weiteren Ausgestaltung der Erfindung wird die Tatsache genutzt, dass die Luft/Wasser-Durchlässigkeit von zwei übereinander liegenden, offenporigen Materialien (Trägerschicht und Abdeckung mit Lüftungsöffnungen oder eine luft- und dampfdurchlässige Faserschicht) unterbrochen wird, wenn sich zwischen diesen, ein quellfähiges Material, beispielsweise ein Absorbergranulat oder ein Thermoplast-Elastomer-Composite, was auch als flaches Material vorliegen kann, befindet, welches im trockenen Zustand einen Luftaustausch durch den Schichtaufbau hindurch ermöglicht. Eine durch Flüssigkeit bedingte Volumenzunahme dichtet die Zwischenräume, die sich zwischen diesen zwei übereinander liegenden Schichten befinden oder die als Kammern ausgebildet sind, wasserdicht ab. Beim Trocknen reduziert sich das Volumen des Dampfabsorbers wieder und Luft kann durch die Zwischenräume zirkulieren. Die zwei übereinander liegenden Materialien verhindern ein Verlieren des Absorbergranulats oder ähnlichen Materials und begrenzen den Raum, der dem Material bei seinem Aufquellen zur Verfügung steht, was eine Verdichtung und damit verbundene Luft- bzw. Wasserundurchlässigkeit zur Folge hat.

Gegebenenfalls kann das quellfähige Material mit einem Füllmaterial ergänzt werden, zum Beispiel Kork- oder Kunststoffgranulat oder -fasern. Als Füllstoff können ferner beispielsweise eingesetzt werden: Polymerverbindungen, Thermoplast-Elastomer-Composite, Kohle, tierische Fasern wie Haare, Daunen, Leder, Knochen, Horn, pflanzliche Fasern wie Baumwolle, Cellulose, Pappe, Leinen, Kokosschalen, Holz, Faser, Kräuter-., metallische, mineralische Materialien, auch in anderen Formen als in Faserform, Carbonfaser-Gewirke, Gummi bzw. Materialien oder Mischungen hieraus, Pulver oder Granulate oder Materialien mit wärmespeichernden Eigenschaften, z.B. mikroverkapselte Wachse.

An dem Rahmen können Befestigungen, wie beispielsweise Verbindungsflächen, Klebeflächen oder Nähte, auf- bzw. angebracht sein, die es ermöglichen, den Belüftungseinsatz über Materialdurchbrüche oder Öffnungen in dem betreffenden Artikel in einer gewünschten Position zu halten. Bei Trockenheit kann so Luft durch den Belüftungseinsatz hindurchzirkulieren. Die Verbindung an den Verbindungsflächen kann beispielsweise durch Magnetverbindung, Klickverbindung, Steckverbindung, mit einem zum Zeitpunkt der Verarbeitung fließfähige Material, Schiebeverbindung, Stülpverbindung, Klebverbindung, Falzverbindung, thermoplastisches Verschweißen oder Nähte hergestellt werden.

Dringt Nässe in den Belüftungseinsatz ein, nimmt der Flüssigkeits-/Dampfabsorber diese auf. Bedingt durch die Flüssigkeitsaufnahme und der damit verbundenen Volumenzunahme, wird der Zwischenraum zwischen Trägerschicht und Abdeckung wasserfest abgedichtet. Wird die Zuführung von Feuchtigkeit unterbrochen, verdunstet die zuvor aufgenommene Flüssigkeit, das Volumen des Dampfabsorbers reduziert sich und Luft kann wieder durch den Belüftungseinsatz hindurch zirkulieren.

Die Quelleigenschaften von Dampfabsorbem, zum Beispiel auf der Basis vernetzten Natriumpolyacrylats oder eines quellfähigen Kunststoffes, sind nahezu unbegrenzt reversibel.

Die Dampfdurchlässigkeit eines Dampfabsorbers lässt sich beispielsweise durch eine Schutzschicht steuern, die aus einem Lack bestehen kann. Dies ist insbesondere dann sinnvoll, wenn ein flächiger, bei Feuchtigkeit quellfähiger Dampfabsorber vorliegt und die Ränder zur Befestigung dienen und nicht mit aufquellen sollen.

Nachfolgend soll die Erfindung anhand von Ausführungsbeispielen näher erläutert werden. In den zugehörigen Zeichnungen zeigen:
- Figur 1: einen Querschnitt durch eine erste nicht erfindungsgemäße Variante eines Belüftungseinsatzes im trockenen und feuchten Zustand,
- Figur 2: einen Querschnitt durch eine zweite nicht erfindungsgemäße Variante eines Belüftungseinsatzes im trockenen und feuchten Zustand,
- Figur 3: eine Draufsicht auf einen Belüftungseinsatz gemäß Figur 2,
- Figur 4: eine Drauf- und Schnittansicht auf einen Belüftungseinsatz für Textilien oder für Schuhe mit verschiebbaren Abdeckungen, jeweils einmal im trockenen und feuchten Zustand,
- Figur 5: einen Querschnitt durch eine dritte nicht erfindungsgemäße Variante eines Belüftungseinsatzes im feuchten und trockenen Zustand,
- Figur 6: einen Querschnitt durch eine vierte nicht erfindungsgemäße Variante eines Belüftungseinsatzes im feuchten und trockenen Zustand,
- Figur 7: eine Aufsicht auf einen großflächigen Belüftungseinsatz,
- Figur 8: ein Anordnungsbeispiel für einen Belüftungseinsatz,
- Figur 9: eine Aufsicht auf einen Belüftungseinsatz mit wabenförmig angeordneten Haltestegen und Schnittansichten im trockenen und feuchten Zustand und
- Figur 10: ein Beispiel für einen erfindungsgemäßen Belüftungseinsatz in einer Einlege- bzw. Schuhsohle.
- Figur 11: ein weiteres Beispiel für einen Belüftungseinsatz in Explosivdarstellung,
- Figur 12: den Belüftungseinsatz nach Figur 11 in der Seitenansicht,
- Figur 13: den Belüftungseinsatz nach Figur 11 in der Draufsicht,
- Figur 14: den Belüftungseinsatz nach Figur 11, eingesetzt in einen Schutzhelm,
- Figur 15: den Belüftungseinsatz nach Figur 11, eingesetzt in einen Schuh,
- Figur 16: einen weiteren Belüftungseinsatz im nicht aktivierten Zustand und
- Figur 17: den Belüftungseinsatz nach Figur 16 im aktivierten Zustand.

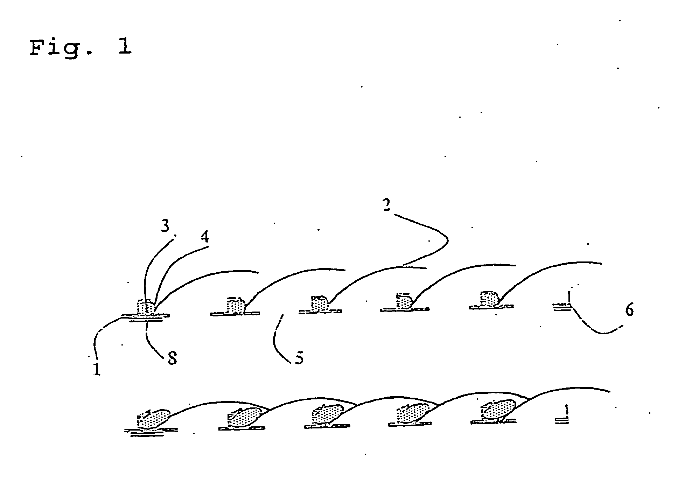

Figur 1 zeigt schematisch einen Belüftungseinsatz mit einer Trägerschicht 1, die später in dem zu belüftenden Artikel nach innen weist und die mit Lüftungsöffnungen 5 versehen ist. Auf der Trägerschicht 1 in Halterungen 6 gehalten sind Hüllen 4, in die ein Dampfabsorber 3 eingeschlossen ist. Mit der Hülle 4 verbunden sind Abdeckelemente 2, und zwar so, dass sie bei Ausdehnung des Dampfabsorbers 3 nach Art eines Scharniers 9 umklappen. Am Rand der Trägerschicht 1 sind Befestigungen 8, zum Beispiel eine Klebefläche, aufgebracht. Die Abdeckelemente 2 sollten aus einem wasserdichten, dampfdurchlässigen Material bestehen.

Bei dieser ersten nicht erfindungsgemäßen Variante wird also die Quellfähigkeit des Dampfabsorbers 3 durch die Flüssigkeitsaufnahme genutzt, um die Abdeckelemente 2 nach unten bzw. oben klappen zu lassen und so die Lüftungsöffnungen 5 zu verschließen und bei der Verdunstung der zuvor aufgenommenen Flüssigkeit durch entsprechende Rückstellkräfte zu öffnen.

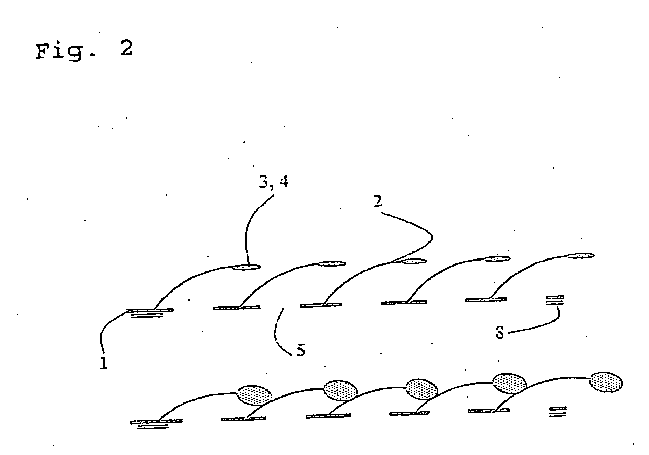

Figur 2 zeigt eine ähnliche Anordnung, nur dass die Hüllen 4 an den nach außen weisenden Enden der Abdeckelemente 2 angebracht sind. Die Ventilwirkung basiert hier auf der Gewichtszunahme des Dampfabsorbers 3, die die Abdeckelemente 2 nach unten oder bei Verdunstung der Flüssigkeit durch eine elastische Rückstellung wieder nach oben klappen lässt.

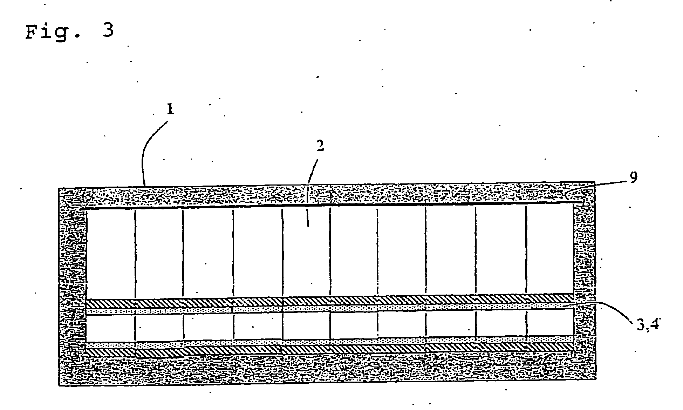

Figur 3 zeigt die Anordnung in einer Ansicht von oben.

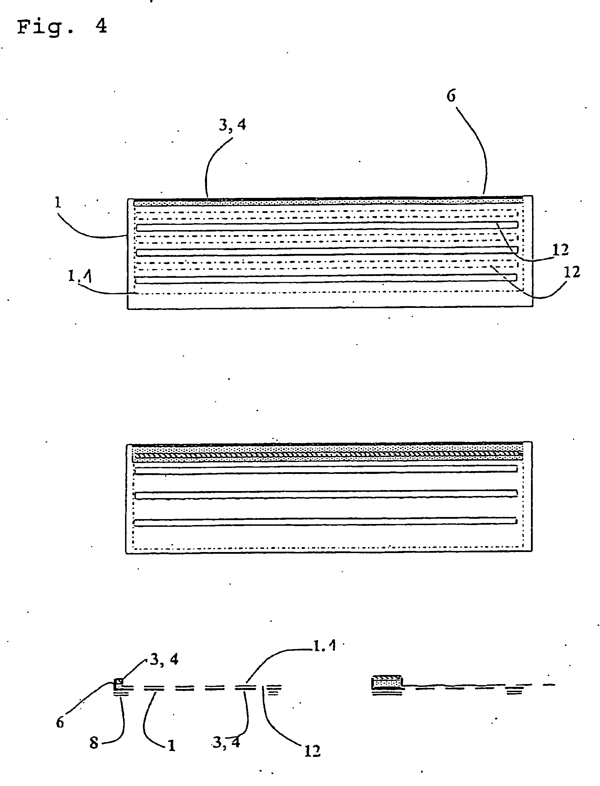

Figur 4 zeigt eine Variante, welche für einen Einsatz an Textilien oder an Schuhoberteilen vorgesehen ist. Hier befindet sich eine Abdeckung 1.1 auf einer Trägerschicht 1, wobei beide jeweils auf gleicher Höhe über Durchtrittsöffnungen 12 verfügen und wenigstens eine der beiden derart angebracht ist, dass sie verschiebbar ist und an wenigstens einer Seite mit dem Dampfabsorber 3 und der Hülle 4 in Verbindung steht. Bedingt durch die Quellung des Dampfabsorbers 3 kann die Abdeckung 1.1 verschoben werden, wodurch sich gleichzeitig die Schnittfläche der Durchtrittsöffnungen 12 verschiebt.

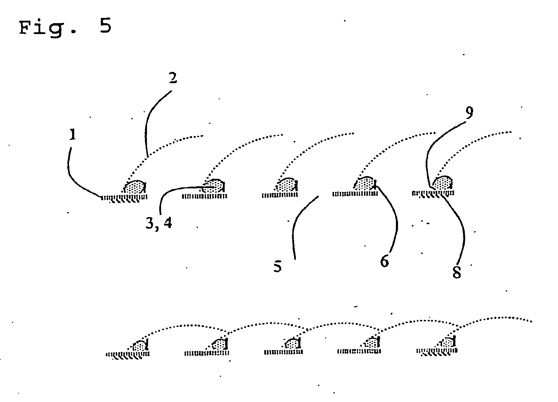

Figur 5 zeigt streifenförmig angeordnete Abdeckelemente 2, an denen sich auf Höhe des Scharniers 9 die Hüllen 4 mit dem Dampfabsorber 3 befinden. Hier werden die Abdeckelemente 2 für einen Flüssigkeitstransport in Richtung des Dampfabsorbers 3 genutzt. Flüssigkeit kann über die Abdeckelemente 2 mit dem Dampfabsorber 3 in Berührung kommen. Für eine schnellere Trocknung des Dampfabsorbers 3 dienen die Abdeckelemente 2 gleichzeitig als Verdunstungsflächen.

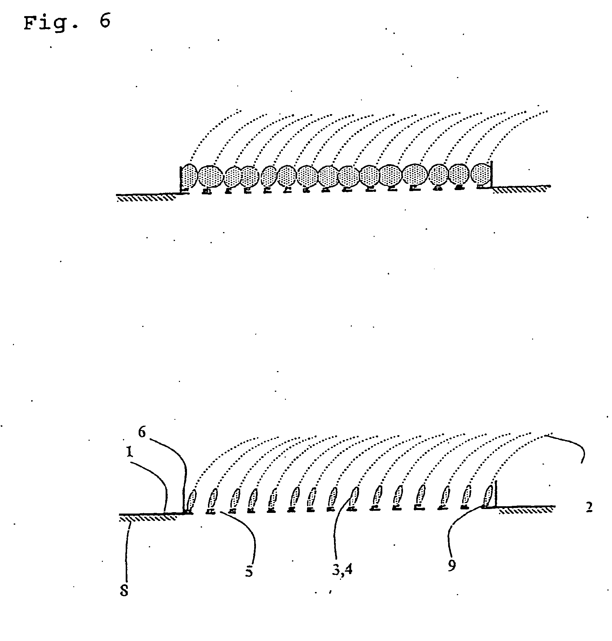

Figur 6 zeigt eine nicht erfindungsgemäße Variante, nach der die Quellfähigkeit des Dampfabsorbers 3 unmittelbar ausgenutzt wird, um die Lüftungsöffnungen 5 in der Trägerschicht 1 zu verschließen. Die Abdeckelemente 2 sind hier mit den Hüllen 4 des Dampfabsorbers 3 verbunden und verhindern ein Befeuchten von außen her.

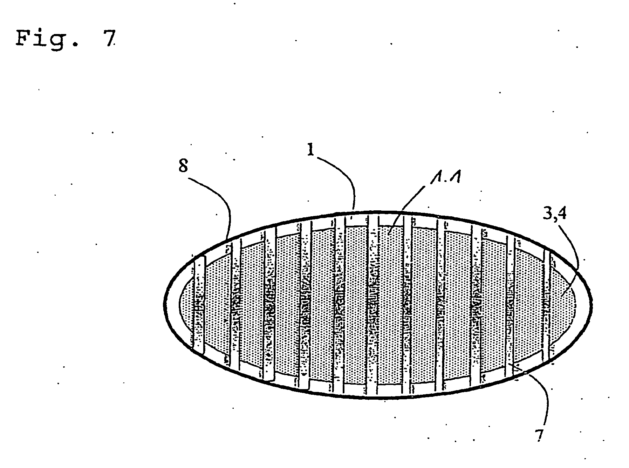

Figur 7 zeigt einen großflächigen Belüftungseinsatz. Um einen zwischen der Trägerschicht 1 und der Abdeckung 1.1 befindlichen Zwischenraum 11 zu begrenzen, wurden hier Haltestege 7 angebracht.

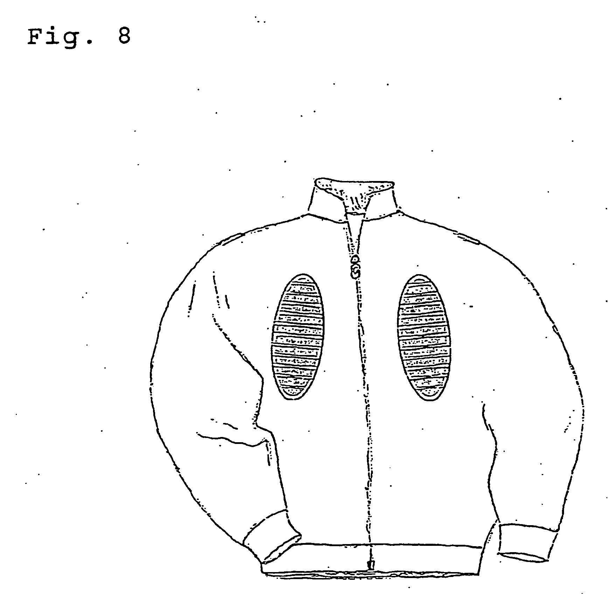

In Figur 8 ist in einem Anordnungsbeispiel die Anbringung eines Belüftungseinsatzes in einer Jacke dargestellt.

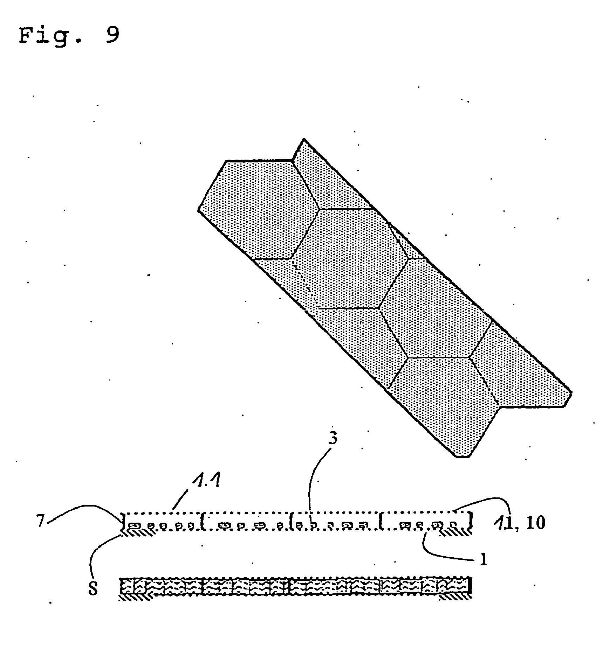

Nach der Variante nach Figur 9 sind Trägerschicht 1 und Abdeckung 1.1 mit wabenförmig angeordneten Haltestegen 7, welche aus dem Material der Trägerschicht 1 durch Materialaussparung ausgebildet sein können, verbunden. Der Verschluss der Lüftungsöffnungen 5 in der Trägerschicht 1 und der Abdeckung 1.1 erfolgt auch hier unmittelbar durch die Quellfähigkeit des Dampfabsorbers 3.

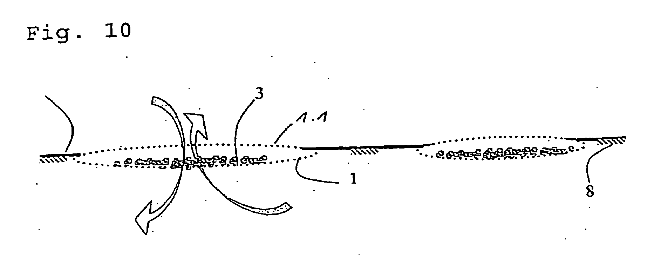

In Figur 10 ist ein Belüftungseinsatz dargestellt, welcher sich innerhalb einer Schuhsohle befindet und vor einem Wassereintritt schützt. In diesem Anwendungsbeispiel bildet die Abdeckung 1.1, bestehend zum Beispiel aus einem nachgiebige Polymer wie TPR oder Nylon, mit der Trägerschicht 1 eine Kapsel, welche über Lüftungsöffnungen 5 verfügt. Zwischen den Abdeckungen 1.1 und der Trägerschicht 1 entsteht ein Zwischenraum 11, in den ein Dampfabsorber 3 eingebracht ist.

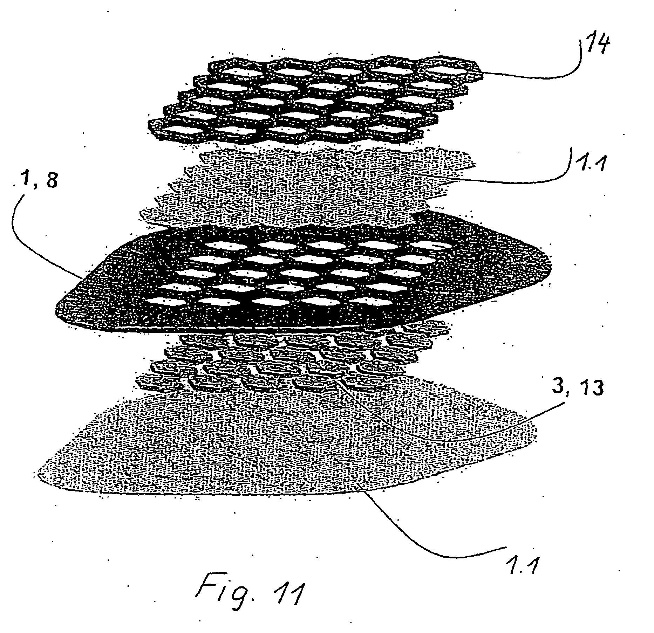

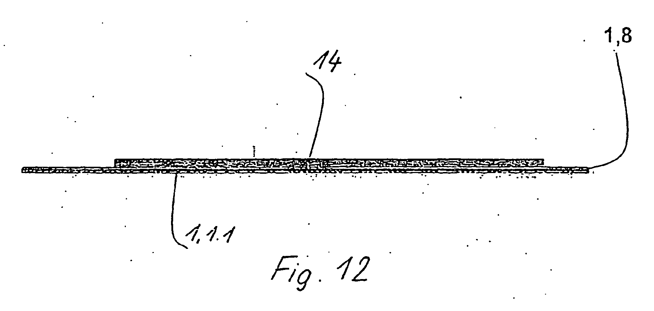

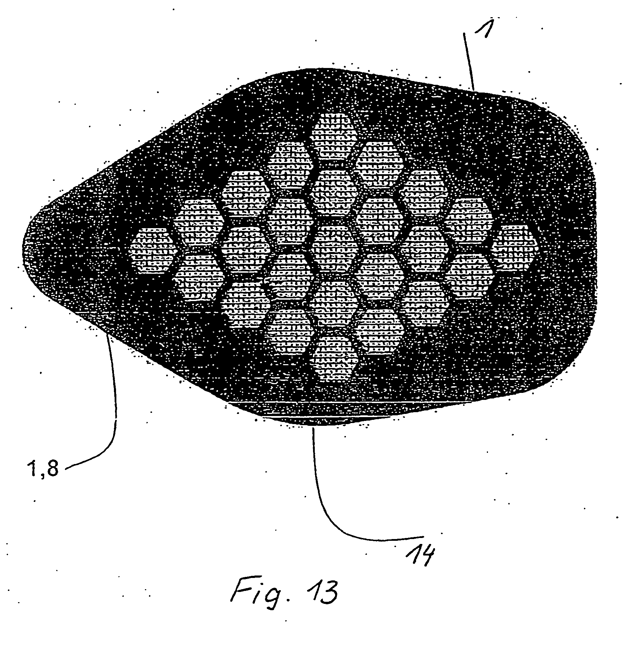

Die Figuren 11 bis 13 zeigen einen Belüftungseinsatz mit einer Trägerschicht 1, die auf beiden Seiten eine Abdeckung 1.1 aus Vlies-Material aufweist, wobei auf die untere Abdeckung 1.1 ein Dampfabsorber 3 aus Thermoplast-Elastomer-Composite zusammen mit einem Füllstoff 13 aufgebracht ist. Als Füllstoff geeignet ist Kork- oder Kunststoffgranulat, wobei für letzteres auch die Verwendung intelligenter Polymere, die auf Wärme reagieren, möglich ist. Nach außen hin befindet sich ein Schutzgitter 14. Das Schutzgitter 14 verhindert eine Beschädigung der Abdeckung 1.1. Es kann durch Spritzgusstechnik hergestellt und durch die Abdeckung 1.1 hindurch mit dem Trägermaterial 1 verbunden sein.

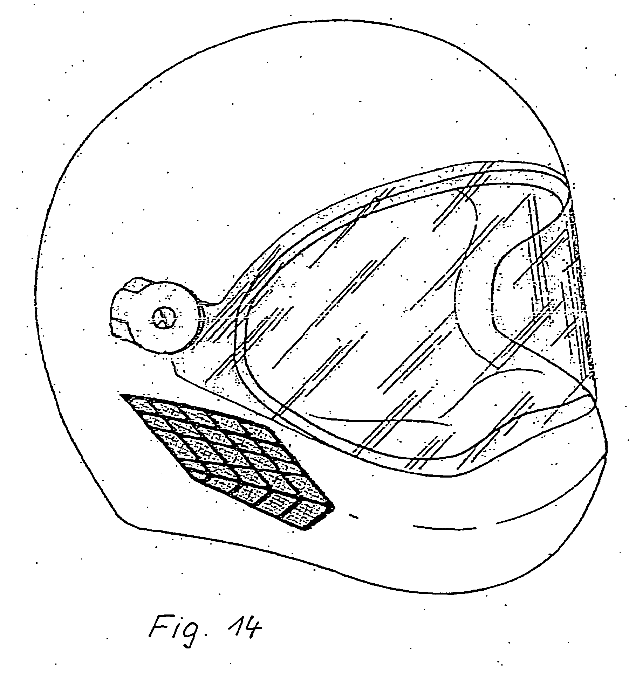

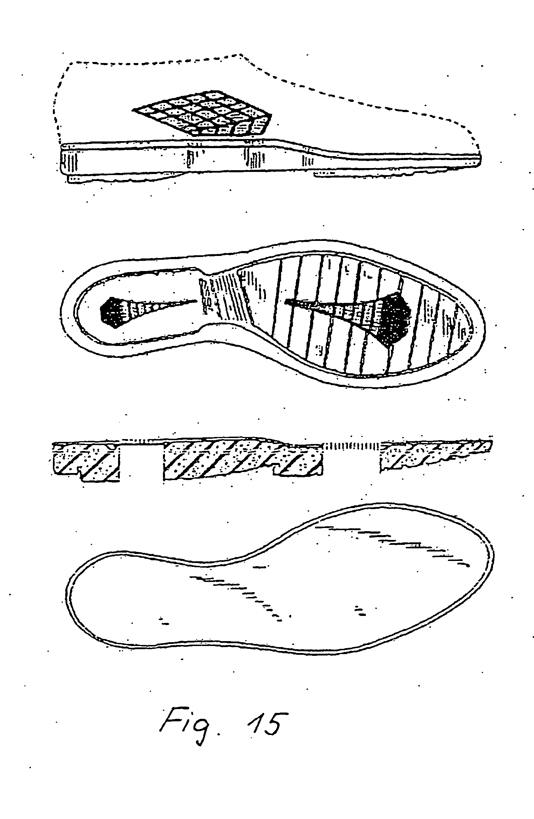

Fig. 14 zeigt den Belüftungseinsatz in der Verwendung in einem Schutzhelm, Fig. 15 in einem Schuh.

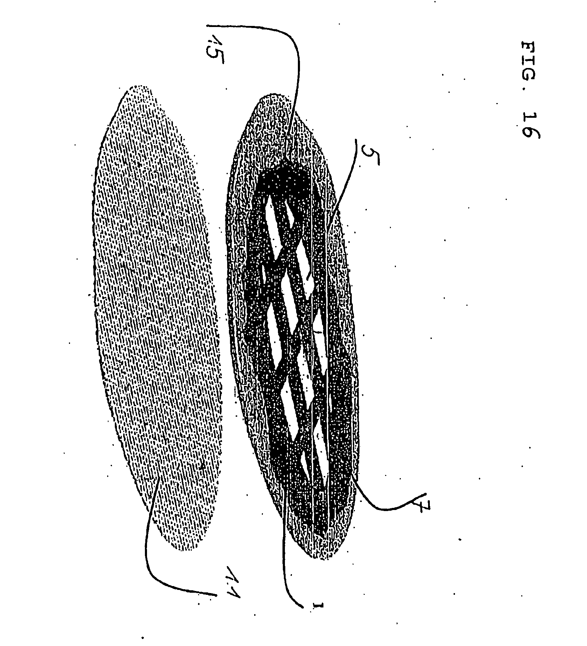

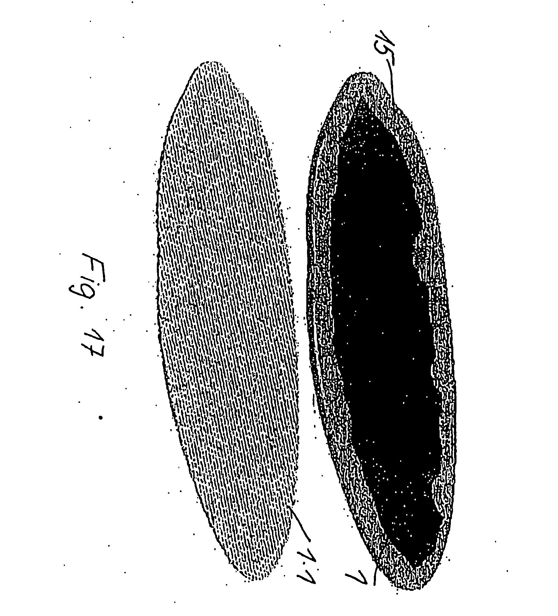

Eine weitere Variante zeigen die Figuren 16 und 17. Hier besteht die gitterförmige Trägerschicht 1 selbst aus dem Dampfabsorber 3, in diesem Fall aus quellfähigem Thermoplast-Elastomer-Composite. Zwischen den das Gitter bildenden Haltestegen 7 sind die Lüftungsöffnungen 5 gebildet. Durch eine Abdeckung 1.1 wird das Eindringen von Schmutz verhindert. Sie dient gleichzeitig als Verdunstungsfläche. Tritt Feuchtigkeit auf die Haltestege 7, quellen diese auf und die Lüftungsöffnungen 5 werden nach und nach verschlossen bis sie wasserdicht sind. Am umlaufenden Rand verbleiben Flächen für Befestigungen 8 an dem zu belüftenden Artikel. Die Quellfähigkeit der Befestigungsflächen wird durch eine wässerfeste Schutzschicht 15 gemindert bzw. ganz unterbrochen.

### Bezugszeichenliste

- 1.: Trägerschicht
- 1.1: Abdeckung
- 2.: Abdeckelement
- 3.: Dampfabsorber
- 4.: Hülle
- 5.: Lüftungsöffnung
- 6.: Halterung
- 7.: Haltesteg
- 8.: Befestigungen
- 9.: Scharnier
- 10.: Faserschicht
- 11.: Zwischenraum
- 12.: Durchtrittsöffnung
- 13.: Füllstoff
- 14.: Schutzgitter
- 15: Schutzschicht

## Patentansprüche

1. Belüftungseinsatz für die Belüftung von Textilien, Schuhen sowie Schuhsohlen, Handschuhen, Schutzhelmen und deren Visieren, Schutzbrillen, Isoliermatten, Polstermatten, Knie- und Schienbeinschützer, Kopfbedeckungen und ähnlichen Artikeln, **dadurch gekennzeichnet, dass**
- eine offenporige Trägerschicht (1) mit einer offenporigen Abdeckung (1.1, 10) versehen ist, die mindestens teilweise aus einem Material (3) bestehen, das durch wenigstens eine Flüssigkeit, Feuchtigkeit oder eine elektrische Spannung eine solche Quell- oder Schrumpfwirkung erfährt oder Formänderung erfährt oder
- ein solches Material (3) so mit der Trägerschicht (1) und/oder, der Abdeckung (1.1, 10) verbunden ist
dass durch die Quell- oder Schmmpfwirkung oder Formänderung ein Freigeben oder Verschließen von Lüftungsöffnungen (5, 12) in der Trägerschicht (1) und/oder der Abdeckung (1.1, 10) erfolgt, wobei die Trägerschicht (1) ein Vliesmaterial ist und die Abdeckung (1.1) ein Vlies-Material oder ein Kunststoff- oder Metallgitter ist.

2. Belüftungseinsatz nach Anspruch 1, **dadurch gekennzeichnet, dass** das Material (3) ein Granulat oder ein granulatförmiger Flüssigkeitsabsorber ist.

3. Belüftungseinsatz nach Anspruch 1, **dadurch gekennzeichnet, dass** das Material (3) stückiges Flachmaterial ist.

4. Belüftungseinsatz nach Anspruch 1, **dadurch gekennzeichnet, dass** das Material (3) fadenförmig geformt ist.

5. Belüftungseinsatz nach einem der vorhergehenden Ansprüche, **dadurch gekennzeichnet, dass** das quellfähige Material (3) ein Dampfabsorber aus vernetztem Natriumpolyacrylat oder einem Polyacrylsäurecopolymer ist.

6. Belüftungseinsatz nach einem der Ansprüche 1 bis 4, **dadurch gekennzeichnet, dass** das formändernde Material (3) eine Formgedächtnislegierung ist.

7. Belüftungseinsatz nach einem der vorhergehenden Ansprüche, **dadurch gekennzeichnet, dass** er mehrfach übereinander aufgebaut ist.

8. Belüftungseinsatz nach einem der vorhergehenden Ansprüche, **dadurch gekennzeichnet, dass** das quellfähige Material (3) in den Zwischenraum (11) zwischen der Trägerschicht (1) und einer Abdeckung (1.1) eingebracht ist.

9. Belüftungseinsatz nach einem der vorhergehenden Ansprüche, **dadurch gekennzeichnet, dass** die Trägerschicht (1) und Abdeckung (1.1) an ihren Rändern durch Verkleben oder thermoplastisches Verschweißen und/oder durch zum Zeitpunkt der Verarbeitung fließfähige Materialien derart miteinander verbunden sind, dass sie einen Rahmen bilden.

10. Belüftungseinsatz nach Anspruch 8 oder 9, **dadurch gekennzeichnet, dass** Trägerschicht (1) und Abdeckung (1.1) durch Haltestege (7) voneinander getrennt sind.

11. Belüftungseinsatz nach Anspruch 10, **dadurch gekennzeichnet, dass** die Haltestege (7) Wabenform aufweisen.

12. Belüftungseinsatz nach Anspruch 10, **dadurch gekennzeichnet, dass** die Haltestege (7) eine Gitterform aufweisen.

13. Belüftungseinsatz nach einem der vorhergehenden Ansprüche, **dadurch gekennzeichnet, dass** an dem Rahmen Befestigungen (8) wie Klebeflächen oder Nähte auf- bzw. angebracht sind.

14. Belüftungseinsatz nach einem der vorhergehenden Ansprüche, **dadurch gekennzeichnet, dass** die Abdeckung (1.1) durch ein Schutzgitter abgedeckt ist.

15. Belüftungseinsatz nach einem der vorhergehenden Ansprüche, **dadurch gekennzeichnet, dass** dem quellfähigen Material (3) ein Füllmaterial (13) beigemischt ist.

16. Verwendung eines Belüftungseinsatzes nach einem der vorhergehenden Ansprüche, für die Belüftung von Textilien, Schuhen sowie Schuhsolen, Handschuhen, Schutzhelmen und deren Visieren, Schutzbrillen, Kopfbedeckungen, Isoliermatten, Polstermatten, Knie- und Schienbeinschützern.

## Claims

1. Ventilation insert for ventilating textiles, shoes and soles of shoes, gloves, protective helmets and their visors, protective goggles, insulating mats, cushioning mats, knee pads and shin pads, headgear and similar articles, **characterised in that**
- an open-pored support layer (1) is provided with an open-pored cover (1.1, 10), which are at least partly composed of a material (3), which undergoes such a swelling or shrinking effect on account of at least one of liquidity, moisture, or an electrical voltage, or undergoes change of form or
- a material of this type (3) is connected to the support layer (1) and/or the cover (1.1, 10) in such a way
that on account of the swelling or shrinking effect or change of form an exposure or closure of ventilation openings (5, 12) in the support layer (1) and/or the cover (1.1, 10) takes place, wherein the support layer (1) is a fleece material and the cover (1.1) is a fleece material or a plastic or metal grid.

2. Ventilation insert according to claim 1, **characterised in that** the material (3) is a granulate or a granulate-shaped fluid absorbent.

3. Ventilation insert according to claim 1, **characterised in that** the material (3) is pieces of flat material.

4. Ventilation insert according to claim 1, **characterised in that** the material (3) is formed from filiform material.

5. Ventilation insert according to any one of the preceding claims, **characterised in that** the material (3) capable of swelling is a vapour absorbent of cross-linked sodium polyacrylate or a polyacrylate acid copolymer.

6. Ventilation insert according to any one of claims 1 to 4, **characterised in that** the form-changing material (3) is a shape memory alloy.

7. Ventilation insert according to any one of the preceding claims, **characterised in that** it is multiply built-up.

8. Ventilation insert according to anyone of the preceding claims, **characterised in that** the material (3) capable of swelling is introduced into the interstice (11) between the support layer (1) and a cover (1.1).

9. Ventilation insert according to any one of the preceding claims, **characterised in that** the support layer (1) and the cover (1.1) are connected to each other on their edges by an adhesive or thermoplastic bonding and/or by materials that flow at the time of manufacture in such a way that they form a fame.

10. Ventilation insert according to claim 8 or 9, **characterised in that** support layer (1) and cover (1.1) are separated from each other by braces (7).

11. Ventilation insert according to claim 10, **characterised in that** the braces (7) have a honeycomb form.

12. Ventilation insert according to claim 10, **characterised in that** the braces (7) have a grid form.

13. Ventilation insert according to any one of the preceding claims, **characterised in that** anchorages (8) such as adhesive surfaces or seams are affixed or attached to the frame.

14. Ventilation insert according to any one of the preceding claims,
**characterised in that** the cover (1.1) is covered by a protective grid.

15. Ventilation insert according to any one of the preceding claims, **characterised in that** a filler (13) is mixed with the material (3) capable of swelling.

16. Use of a ventilation insert according to any one of the preceding claims, for ventilating textiles, shoes and soles of shoes, gloves, protective helmets and their visors, protective goggles, headgear, insulating mats, cushioning mats, knee pads and shin pads.

## Revendications

1. Insert d'aération pour l'aération de textiles, de chaussures et de semelles, de gants, de casques et de leurs visières, de lunettes de protection, de panneaux isolants, de panneaux de rembourrage, de protections du genou et du tibia, de couvre-chefs et d'articles similaires, **caractérisé par le fait que**
- une couche support (1) à pores ouverts est munie d'un revêtement (1.1, 10) à pores ouverts qui est constitué au moins en partie d'un matériau (3) qui, sous l'action d'au moins un liquide, de l'humidité ou d'une tension électrique, subit un effet de gonflement ou de retrait ou une déformation tels, ou
- un tel matériau (3) est relié à la couche support (1) et/ou au revêtement (1.1, 10) de manière telle
que l'effet de gonflement ou de retrait ou la déformation provoque le dégagement ou l'obturation d'orifices d'aération (5, 12) dans la couche support (1) et/ou le revêtement (1.1, 10), la couche support (1) étant un matériau non tissé, et le revêtement (1.1) étant un matériau non tissé ou une grille en matière plastique ou en métal.

2. Insert d'aération selon la revendication 1, **caractérisé par le fait que** le matériau (3) est un produit granulé ou un absorbant de liquide en forme de granulés.

3. Insert d'aération selon la revendication 1, **caractérisé par le fait que** le matériau (3) est un matériau plat en morceaux.

4. Insert d'aération selon la revendication 1, **caractérisé par le fait que** le matériau (3) est filiforme.

5. Insert d'aération selon une des revendications précédentes, **caractérisé par le fait que** le matériau (3) apte à gonfler est un absorbant de vapeur constitué de polyacrylate de sodium réticulé ou d'un copolymère d'acide polyacrylique.

6. Insert d'aération selon une des revendications 1 à 4, **caractérisé par le fait que** le matériau (3) déformable est un alliage à mémoire de forme.

7. Insert d'aération selon une des revendications précédentes, **caractérisé par le fait qu'**il est superposé plusieurs fois.

8. Insert d'aération selon une des revendications précédentes, **caractérisé par le fait que** le matériau (3) apte à gonfler est placé dans l'espace intermédiaire (11) entre la couche support (1) et un revêtement (1.1).

9. Insert d'aération selon une des revendications précédentes, **caractérisé par le fait que** la couche support (1) et le revêtement (1.1) sont liés l'un à l'autre sur leurs bords, par collage ou soudage thermoplastique et/ou par des matériaux qui sont fluides au moment de leur mise en oeuvre, de telle sorte qu'ils forment un cadre.

10. Insert d'aération selon la revendication 8 ou 9, **caractérisé par le fait que** la couche support (1) et le revêtement (1.1) sont séparés l'un de l'autre par des entretoises (7).

11. Insert d'aération selon la revendication 10, **caractérisé par le fait que** les entretoises (7) présentent une forme alvéolaire.

12. Insert d'aération selon la revendication 10, **caractérisé par le fait que** les entretoises (7) se présentent sous forme de grilles.

13. Insert d'aération selon une des revendications précédentes, **caractérisé par le fait que** des moyens de fixation (8), tels que des surfaces adhésives ou des coutures, sont placés sur le cadre.

14. Insert d'aération selon une des revendications précédentes, **caractérisé par le fait que** le revêtement (1.1) est recouvert d'une grille de protection.

15. Insert d'aération selon une des revendications précédentes, **caractérisé par le fait qu'**un matériau de remplissage (13) est mélangé au matériau (3) apte à gonfler.

16. Utilisation d'un insert d'aération selon une des revendications précédentes, en vue de l'aération de textiles, de chaussures et de semelles, de gants, de casques et de leurs visières, de lunettes de protection, de couvre-chefs, de panneaux isolants, de panneaux de rembourrage, de protections du genou et du tibia.
